# EUROPEAN PATENT SPECIFICATION

(11) **EP 2 725 990 B1**
(45) Date of publication and mention of the grant of the patent: **30.05.2018**
(21) Application number: 12731845.9
(22) Date of filing: 19.06.2012
(51) Int. Cl.: A61B 17/34

(54) **SYSTEM FOR FIDUCIAL DEPLOYMENT**
SYSTEM FÜR DEN EINSATZ VON BEZUGSMARKERN
SYSTÈME POUR DÉPLOIEMENT DE REPÈRE

(30) Priority: 28.06.2011 US 201161502132 P
(43) Date of publication of application: 07.05.2014
(73) Proprietor: Cook Medical Technologies LLC, Bloomington, IN 47404 (US)
(72) Inventor: MCHUGO, Vincent, Birdhill Co. Tipperary (IE); CLANCY, Michael, Monaleen Co. Limerick (IE); NEILAN, John, Gort Co. Galway (IE)
(74) Representative: Mathys & Squire LLP
(86) International application number: PCT/US2012/043146
(87) International publication number: WO 2013/003119

(56) References cited:
- US-A1- 2003 013 934
- US-A1- 2005 038 355
- US-A1- 2008 287 782
- US-A1- 2011 152 611

## Description

### CROSS-REFERENCE TO RELATED APPLICATIONS

This application claims priority to U.S. Patent No. US2013006101, filed June 28, 2011.

### TECHNICAL FIELD

The invention relates generally to a medical device system including one or more fiducials. More particularly, the invention pertains to specially-configured fiducials, needles configured for use with them.

### BACKGROUND

Medical procedures often require locating and treating target areas within a patient. Focused, dose-delivery radiation therapy requires locating the target with a high degree of precision to limit damaging healthy tissue around the target. It is particularly important to know or estimate the precise location of the target in radiation oncology because it is desirable to limit the exposure of adjacent body parts to the radiation in a patient already suffering the depredations of cancer. However, in all treatment procedures, whether radiologic or otherwise, it is most desirable to be able to accurately target a region to be treated.

In many applications, it is not possible to directly view a treatment target or portion thereof (such as, for example, a cancerous tumor, cyst, pseudocyst, or other target) that needs to be acted on in some manner. As one example, when treating a lung or pancreatic tumor with radiation, it may not be possible to view the actual tumor within the patient immediately before the radiation treatment. It is therefore highly advantageous to have some mechanism for permitting the tumor to be located accurately so that the radiation treatment can be targeted at the tumor while avoiding damage to healthy tissue.

Even for target regions that may be visualized using CAT (computer-assisted tomography) scans, MRI (magnetic resonance imaging), x-rays, ultrasound, or other techniques, difficulties often arise in targeting a treatment. This is particularly true for target regions within a torso of a patient and soft tissue regions. Due to the mobility of tissues in those regions (e.g., movement of internal organs during respiration and/or digestion, the movement of breast tissue with any change of body position), a target region may not remain fixed relative to anatomical landmarks and/or to marks that can be placed onto an external surface of a patient's body during one of those visualization procedures.

Several techniques have been developed to address this problem. One such technique is to place markers into the patient along the margins of the target region. The markers may be active (e.g., emitting some kind of signal useful in targeting a therapy) or passive (e.g., non-ferromagnetic gold markers - called fiducials - that can be used for targeting under ultrasound, MRI, x-ray, or other targeting techniques, which may be included in a treatment device).

A fiducial is typically formed of a radio-opaque material so that the target can be effectively located and treated with a device that targets a site using the fiducials as positional markers under radiographic detection. Typically, the fiducials may be inserted into the patient during a simple operation. Percutaneous placement is most commonly used. However, use of minimally-invasive placement via an endoscope has recently developed for fiducial placement into a patient's internal organs. For example, percutaneous placement of fiducials along the margins of a pancreatic tumor can be complex and painful (particularly for obese patients, where the needle size is necessarily larger). Another process using percutaneously implanted objects in a patient is brachytherapy. In brachytherapy, radioactive sources or "seeds" are implanted into and/or adjacent a tumor to provide a high dose of radiation to the tumor, but not the healthy tissue surrounding the tumor.

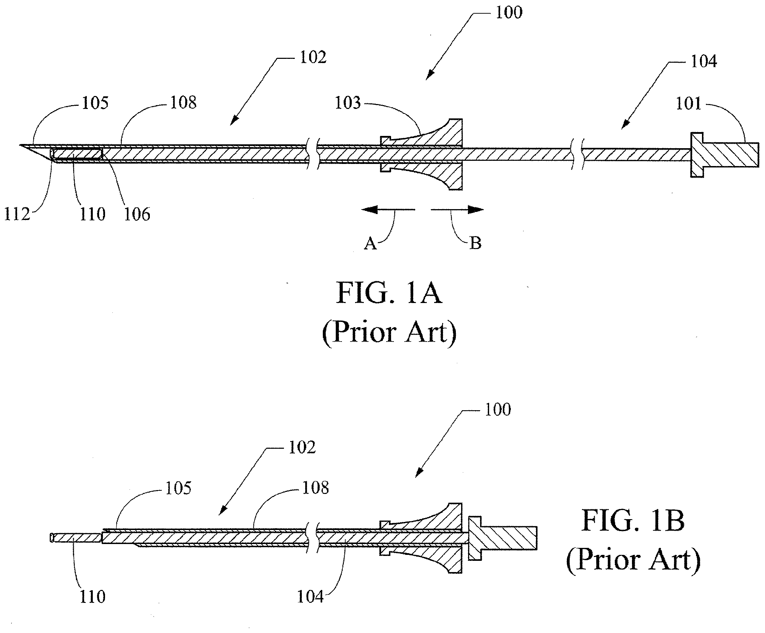

FIGS. 1A and 1B show longitudinal sectional views of a two-piece introducer 100 of the prior art useful for placement of brachytherapy seeds or fiducials. Referring first to FIG. 1A, the introducer 100 includes a needle 102 and a stylet 104 slidably disposed within the needle 102. The stylet 104 includes a first handle 101 and a blunt distal end 106. The needle 102 includes a second handle 103 and a bevel-tipped cannula 108 extending through the second handle 103. The cannula 108 is configured to hold a seed/fiducial 110. The cannula 108 has a distal tip 105 configured for percutaneous implantation of the seed/fiducial 110 into the patient.

In a "pre-loaded configuration," the seed/fiducial 110 is retained in the cannula 108 by a plug 112 made from bone wax or other suitable bio-compatible material(s). This is typically accomplished by a "muzzle-loading" technique where the fiducial is placed into the distal end of the needle and then held in place by the bone wax plug. This can present some challenges, as the bone wax plug 112 can be visible as an artifact in the patient, potentially interfering with clear visualization of body structures or treatment devices. With this configuration, the cannula 108 must be withdrawn and reloaded after delivery of each seed/fiducial 110. If the target locations for the fiducials are very far apart, use of a single percutaneous introducer cannula/trocar for multiple introductions of the cannula 108 may not be possible. In such a circumstance, the patient must endure several percutaneous punctures (and the increased attendant risk of infection for each).

To implant the desired arrangement of seeds/fiducials 110 at a target location in a patient, an operator pushes the cannula 108 in a first direction (arrow A) to insert the tip 105 into the patient (typically under fluoroscopic visualization). The operator then pushes the second handle 103 further in the first direction to position the tip 105 at the desired depth within the patient where a seed/fiducial 110 is to be implanted. Throughout this motion, the operator moves the needle 102 and the stylet 104 together as a unit. At the desired depth/location, the operator grasps the first handle 101 with one hand and the second handle 103 with the other hand. Then, the operator holds the first handle 101 stationary while simultaneously sliding the second handle 103 back in a second direction (arrow B) toward the first handle 101. As shown in FIG. 1B, this movement causes the cannula 108 to retract over the seed/fiducial 110 to implant it in the patient. Alternatively, the operator may move the first handle 101 in the first direction (arrow A) while sliding the second handle 103 back in the second direction (arrow B) or holding it stationary. This causes the stylet 104 to push the seeds 110 out of the cannula 108. The procedure is then repeated to place other seeds/fiducials 110. When being used for targeting of radiation therapy, a minimum of three fiducials is typically required.

As will be appreciated from the disclosed structure, after deploying one fiducial, one may alternatively reload the introducer 100 from the proximal end by completely withdrawing the stylet 104, then placing another fiducial into the needle lumen and advancing it therethrough to a second location to which the distal needle tip 105 has been directed (a "breech-loading" technique). Provided that the fiducial target sites are sufficiently close together to allow this technique, it can reduce the number of percutaneous punctures or other access procedures needed to place more than one fiducial. However, it creates a problem for procedures where ultrasound is being used or is to be used in the near-future because it introduces air pockets into the tissue and related fluids. Those air pockets with tissue and/or fluid are echogenic in a manner that can interfere with ultrasound visualization of a target area and/or tools being used to diagnose or treat in/around the area. In some brachytherapy techniques, a series of fiducials may be preloaded into the needle - either separately or connected by a suture or similar device - then placed together in fairly close proximity; however, such a technique typically is not effective for placing three or more fiducials in sufficiently disparate locations to use for targeting a treatment relative to, for example, margins of a tumor.

The process is similar when implemented endoscopically in the manner developed rather recently, except that the needle and stylet are of the type known in the art for use through the working channel of an endoscope. One limitation of current endoscopic techniques is the size of fiducial that can be introduced. With the size limitation of endoscope working channels, the largest needle that can typically be used without risking bending, crimping, curving or otherwise damaging a needle (that does not have an internal stylet or other support) during advancement out of the endoscope to an anatomical target is a 19-gauge needle. This limits the size of the fiducial that can be introduced through the needle lumen using current, cylindrical fiducials. The endoscopic technique generally suffers from the same reloading problems as described above. Even though the external percutaneous punctures are not an issue, having to withdraw and reload takes up valuable time and complicates the procedure, potentially requiring additional personnel, whether only the stylet is withdrawn for "breech-loading" or the entire device is withdrawn for "muzzle-loading."

It would be desirable to use ultrasound, and particularly endoscopic ultrasound (EUS) for navigation and placement of fiducials. As such it would be desirable to provide and use the largest possible fiducial that will provide improved echogenicity based on its size and echogenic profile. It would be desirable to provide multiple fiducials in a needle that can be introduced in a controlled serial manner (one at a time) rather than requiring manual reloading after placement of each fiducial.

Reference is directed to US 2011/0152611 which forms the basis of the preamble of claim 1, and discloses a fiducial deployment system with a handle configured for actuation of same. A fiducial may include one or more protuberances configured to engage one or more slots in a needle of the system. The needle may be configured to deliver a plurality of fiducials to a target location in serial fashion, one at a time. In certain embodiments, echogenic placement of fiducials may present certain advantages. The handle may include structures configured for incrementally or otherwise controlledly deploying one or more fiducials by advancing a stylet through and/or retracting the body of a needle in which fiducials are disposed.

### BRIEF SUMMARY

The present invention is defined in the appended claims, whereby claim 1 defines the invention and the dependent claims disclose the preferred emebodiments. Embodiments of a fiducial deployment system described herein may include one or more of a needle including a dimpled retention means configured to releasably retain a plurality of fiducials, each of which is equipped with a compressible portion, where the compressible portion is configured to retain the fiducial until it is advanced to the dimpled retention means and sufficiently compressed to pass thereby and be deployed.

### BRIEF DESCRIPTION OF THE DRAWINGS

FIGS. 1A-1B show a prior art fiducial introducer and method of use;
FIG. 2 shows one embodiment of a fiducial deployment system; and
FIGS. 2A-2C show embodiments of fiducials useful with the system of FIG. 2.

### DETAILED DESCRIPTION

The terms "proximal" and "distal" are used herein in the common usage sense where they refer respectively to a handle/doctor-end of a device or related object and a tool/patient-end of a device or related object. Certain embodiments may be configured for placement of fiducials through an endoscope. More particularly, in certain embodiments, a fiducial placement system may be configured of sufficient length and flexibility for use and actuation through a working channel of a gastrointestinal endoscope providing access to the gastrointestinal tract and/or adjacent structures in a patient body.

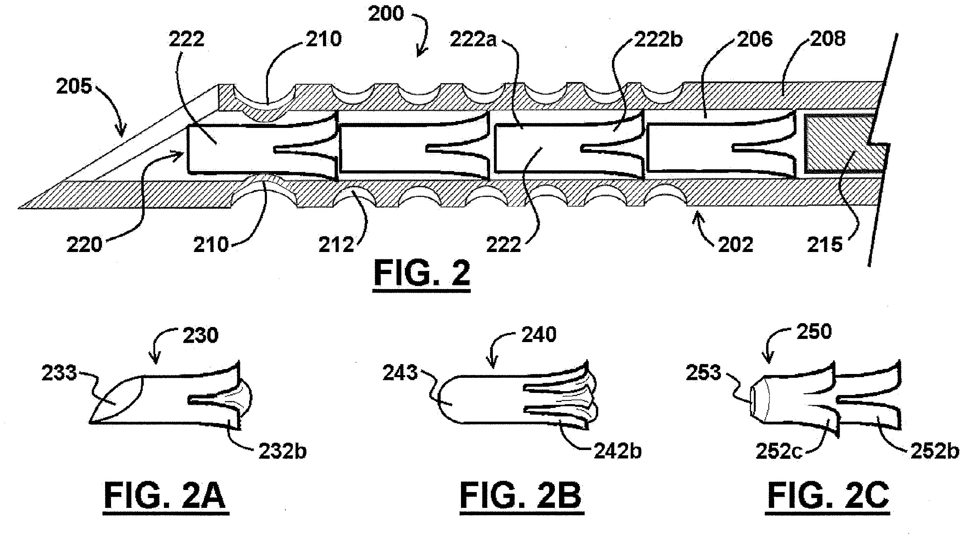

A fiducial deployment system 200 is described with reference to FIG. 2. The system 200 includes a needle 202 that includes a generally tubular cannula body 208. The cannula 208 in this embodiment includes a beveled distal tip 205 configured for penetrating tissue and directing the cannula's contents to a target location, but other embodiments may include non-beveled tips or other designs. The body 208 defines a needle lumen 206 that extends longitudinally through at least a lengthwise portion of the cannula body to a distal needle end opening 207.

An outer surface of the cannula body 208 is dimpled to enhance its ability to reflect ultrasound waves and thereby provide a desirable echogenic profile. This dimpled characteristic may be embodied as a different irregular, patterned, or textured surface feature (e.g., knurled, ribbed) that may enhance the echogenicity of the cannula 208, which will aid in visualizing it during EUS-guided placement, and allow it to be used in ultrasound visualization of a target site (e.g., a tumor) being marked by one or more fiducials. Other echogenic enhancements may be provided in addition to, or instead of, the dimpling. For example, certain echogenic polymers may be used in the cannula construction, or as a coating of a metal cannula. Other echogenic enhancements known in the art may be implemented within the scope of the claims. The dimpled or otherwise echogenically-enhanced region preferably will include a distal needle end region, while a more proximal length of the needle may be free of dimples and/or other echogenicity-enhancing features.

At least one substantially inflexible detent 210 is provided as a protrusion extending radially in the needle lumen 206. The detent here is embodied as a pair of deeper dimples 212 that provide a restricted region of smaller internal diameter for the needle lumen 206 than is provided for the inner diameter of the lumen's major length proximal of the detent 210. In other embodiments, the detent 210 may not include an externally visible dimple, and/or it may include one, two, three, or more protrusions into the needle lumen 206.

At least one fiducial, embodied here as a plurality of fiducials 220 (which is a preferred embodiment) is included in the needle lumen 206. Each of the fiducials 220 includes a generally columnar body 222 slidably disposed in the needle lumen 206. The body 222 may be substantially solid, substantially hollow, or otherwise configured in any manner appropriate for providing a desirable fluoroscopic identifiability of the fiducial 220. Fiducials 220 (and other fiducial embodiments described below) preferably will be formed of a radio-opaque, non-ferromagnetic material such as, for example, gold, platinum, palladium, iridium, tantalum, or alloys thereof, with one preferred embodiment including an alloy of palladium with rhenium (advantages of which may include desirable radio-opacity, market-price stability superior to gold, and ultrasound-reflectivity/echogenicity due to density). Being radio-opaque will allow the fiducial to be used in deployment techniques using fluoroscopy, as well as making it detectible/ visualizable by radiographic means during a treatment or other procedure where it may be desirable to know the location(s) of one or more fiducials. Being non-ferromagnetic will lessen the likelihood that visualization techniques or other procedures employing magnetic fields such as, for example, MRI, will re-orient or otherwise dislodge a fiducial. Echogenic construction of a fiducial or needle may be enhanced by surface texture, but can also be provided by structural inclusions such as embedded bubbles or beads that provide for a different ultrasound reflectivity than material surrounding them. Fiducials may also be coated with a material (e.g., parylene) configured to reduce backscatter during radiography.

The generally columnar body 222 of each fiducial 220 includes a distal first lengthwise portion 222a having a first outer diameter. That first outer diameter preferably is the same as, or slightly less than the inner diameter provided by the detent(s) 210, such that the fiducial 220 can be advanced distally past the detent(s) 210. The distal end of the fiducial 220 may include a blunt cylindrical face as shown in FIG. 2. Other embodiments may include a radiused edge (as shown in the fiducial embodiment of FIG. 2C), a domed or otherwise rounded distal end (as shown in the fiducial embodiment of FIG. 2B), an angled distal end that may or may not be configured to parallel a bevel angle of the distal needle end 205 (as shown in the fiducial embodiment of FIG. 2A), or another distal end configuration suitable for passage out of the needle 202.

The generally columnar body 222 of each fiducial 220 includes a proximal second lengthwise portion 222b that is splayed to provide a second outer diameter. The second outer diameter preferably is the same as, or slightly less than the inner diameter provided by the needle lumen 206 proximal of the detent(s) 210. Also, the second outer diameter preferably is the same as, or slightly greater than the restricted inner diameter provided by the needle lumen 206 portion at detent(s) 210. This construction provides for capture and retention of the splayed proximal fiducial portion 222b. The fiducials 220 shown in FIG. 2 include a single transverse split or cut across the width of each fiducial's proximal end that extends longitudinally into the body to provide the proximal splayed portion 222b. As shown, the widest part of the splayed fiducial portion forms a portion of a larger outer diameter (as compared to the distal body 222a) that frictionally but slidably engages the wall of the needle lumen 206. The frictional engagement of the outer diameter of the splayed portion 222b preferably is sufficiently strong to retain the fiducial 220 within the needle lumen 206, but configured to allow it to be slidably advanced by, for example, a pushing stylet. In embodiments that include one or more detents 210, the detents may function as a capturing stop for each fiducial.

With the materials contemplated for fiducial construction, the proximal splayed portion 222b preferably will include at least limited flexibility. Specifically, in preferred embodiments, the proximal splayed portion 222b will be sufficiently flexible that its second outer diameter will be radially compressible enough to be advanced distally past the detent(s) 210. In one exemplary embodiment each fiducial 220 may be about 0.12 inches (3.05 mm) long and have a maximum OD (around the splayed region) of about 0.034 inches (0.86 mm). Those of skill in the art will appreciate that appropriate materials for fiducial construction will, at this type of scale/size, have sufficient flexibility for the structure and function described herein.

Other fiducial embodiments may be practicable with the structures described herein. In addition to the example of a fiducial 220 shown in FIG. 2, other examples of fiducial embodiments are shown in FIGS. 2A-2C. Those of skill in the art will appreciate that variants of these and other embodiments may be practiced within the scope of the claims. FIG. 2A shows a fiducial 230 with a tripartite proximal splayed portion 232b, and a beveled distal leading end 233. FIG. 2B shows a fiducial 240 with a blossomed multipart proximal splayed portion 242b and a domed distal leading end 243. The shape of the distal leading end may provide a certain ease or advantage in advancing the fiducial(s) past a detent protrusion. For example, a fiducial with a blunt cylindrical distal end may be more difficult to advance toward and past a detent protrusion 210 than a fiducial with a radiused, chamfered, rounded, beveled, or otherwise shaped distal end configured to ease passage across said protrusion.

FIG. 2C shows a fiducial 250 that includes a plurality of splayed portions, embodied here as a proximal splayed portion 252b, an intermediate splayed portion 252c, and a distal leading end 253 that is generally shaped as the end of a cylinder with a radiused edge. This plurality of splayed portions may provide a user with added control over fiducial deployment. It may also provide enhanced visualizability of a fiducial under ultrasound and/or fluoroscopic imaging. In some embodiments, one of the plurality of splayed portions (e.g., 252c) may be generally longitudinally aligned another of the plurality of splayed portions (e.g., 252b). It should be appreciated that the particular shapes, surface positions on fiducial bodies, and general proportions of these and the other protuberances disclosed herein may be interchanged or otherwise modified within the scope of the claims, including with reference to other fiducial designs such as those disclosed in U.S. Pat. Pub. Nos. 2010/0280367 and 2011/0152611. The generally columnar construction of fiducials described herein includes a broad variety of potential geometries within the scope of the claims, including for example regular and irregular geometric shapes having rounded and/or polyhedral borders and cross-sections. In one preferred embodiment at least one fiducial will include a generally cylindrical body portion.

It will be appreciated that the splayed construction described herein will allow fiducials to be advanced distally out of the needle 202 serially, one at a time, in a controlled manner. A stylet 215 is provided, extending longitudinally through the needle lumen 206. An exemplary method of using a fiducial deployment system may include having an endoscope is provided, including a working channel. In one preferred method, the endoscope may be an EUS endoscope including a distal ultrasound array configured for ultrasound imaging. An endoscope may also include a video element (e.g., CCD, optical camera, or other means for optical visualization). A method of use may include placing fiducials at the margins of a tumor in a patient's pancreas. As such, an appropriate needle body will be of sufficient length and navigability (e.g., pushability and flexibility) to perorally be directed through a patient's gastrointestinal tract to a target site, including doing so via a working channel of an endoscope such as a gastric endoscope, colonoscope, anuscope, or other visualization/procedure-assisting device.

In one aspect, a fiducial deployment may be accomplished by positioning the distal needle end 205 and a fiducial 220 therein at a first target, then retracting the needle 202 while retaining the position of the stylet 215 such that the distal-most fiducial 220 passes the detent 210, exits the needle 202, and remains in a desired first target position. In another aspect, a fiducial deployment may be accomplished by positioning the distal needle end 205 and the distal-most fiducial 220 therein adjacent a first target, then holding the needle 202 in position while advancing the stylet 215 such that the fiducial 220 is advanced past the detent, out of the needle end 205, and into a desired first target position.

A handle (not shown) may be provided that will provide tactile, auditory, and/or visual indicia regarding deployment of a fiducial. It should be appreciated that, when the stylet 215 is advanced distally, the smaller outer diameter distal portion 222a of the fiducial will slide generally easily past the detent 210. However, a user will likely encounter some resistance as the proximal splayed portion 222b engages the detent 210. When the needle end 205 is in a desired position, the user can advance the stylet 215, and thereby the distal-most fiducial, past the detent 210 into the target site. The stylet 215 and the next fiducial in line will be stopped when the proximal splayed portion of that more-proximal fiducial engages the detent 210. The needle may then be repositioned without having to be fully withdrawn from the patient, and the next fiducial deployed in the same manner. Although four fiducials are shown in FIG. 2, it should be appreciated that more or fewer may be provided in a pre-loaded needle.

It will often be preferred that the fiducials (and the splayed portions thereof) be proportioned such that complete deployment of a distal-most fiducial will include it substantially clearing the distal needle tip 205 and will coincide with the proximal-most splayed portion 222b of the next distal-most fiducial contacting the detent(s) 210.

As noted above, after deploying a distal-most fiducial, the user may retract the needle 202 from the first target site, and then direct it to a second target site, where the procedure described above may be repeated. These steps may be repeated for placement of third, fourth, and further fiducials. As is known in the art, these fiducials may be used for "positive targeting" and/or "negative targeting" of a therapy such as radiation therapy ("positive targeting" indicating "treat here", and "negative targeting" indicating "do not treat here"). The present system presents numerous advantages. For example, consider a patient already undergoing an endoscopy procedure to biopsy a located but undiagnosed tissue mass. The endoscopic biopsy can be taken and a tissue slide prepared immediately. If a diagnosis is made (in conjunction with whatever other data are available and pertinent) that the tissue mass will benefit from a treatment where placement of fiducials is indicated, the physician can immediately deploy fiducials in the manner described above, using the same endoscope already positioned for the biopsy.

Drawings and particular features in the figures illustrating various embodiments are not necessarily to scale. Some drawings may have certain details magnified for emphasis, and any different numbers or proportions of parts should not be read as limiting, unless so-designated by one or more claims. Those of skill in the art will appreciate that embodiments not expressly illustrated herein may be practiced within the scope of the present invention, including that features described herein for different embodiments may be combined with each other and/or with currently-known or future-developed technologies while remaining within the scope of the claims presented here. For example, a needle and fiducials of the present system may be used percutaneously, including in another minimally invasive surgical procedure, such as a laparoscopic-type procedure, within the scope of the claimed invention. For example, a target site may be a location in or near the gastrointestinal tract (e.g., liver, pancreas) such as those locations that may be accessible by endoscopy (using a minimally invasive endoscope introduced through a natural patient orifice, e.g., mouth, anus, vagina). This includes -more broadly- sites reachable through NOTES (natural orifice translumenal endoscopic surgery) procedures. The present exemplary method and device may also be used with other minimally-invasive surgical techniques such as percutaneous endoscopic procedures (e.g., laparoscopic procedures) or percutaneous non-endoscopic procedures, but most preferably is used with less invasive endoscopy procedures. It is therefore intended that the foregoing detailed description be regarded as illustrative rather than limiting. And, it should be understood that the following claims, including all equivalents, are intended to define the scope of this invention.

## Claims

1. A fiducial deployment system (200) comprising:
a needle (202) including
a generally tubular cannula body (208) defining a needle lumen (206) disposed through at least a lengthwise portion of the cannula body and
a distal needle end region, the distal end region comprising
a distal needle end opening at a distal end of the needle lumen; and
at least one protrusion configured as a substantially inflexible detent (210);
at least one fiducial (220) comprising
a generally columnar body (222) slidably disposed in the needle lumen and including:
a first lengthwise portion (222a) with a first outer diameter;
a second lengthwise portion (222b) that is splayed with a
second outer diameter greater than the first outer diameter; and
a stylet (215) extending through a portion of the needle lumen and configured to advance the at least one fiducial past the at least one protrusion and out of the distal needle end opening;
**characterised by**:
said protrusion extending radially into the needle lumen to form a restricted inner diameter that is less than an inner diameter of a major length of the needle lumen;
where the first outer diameter of the fiducial is substantially the same as or less than the restricted inner needle diameter adjacent the at least one protrusion; and where the second outer diameter of the fiducial is substantially the same as or less than the inner diameter of the major length of the needle lumen.

2. The fiducial deployment system of claim 1 , where the at least one splayed portion of the at least one fiducial comprises a plurality of splayed portions.

3. The fiducial deployment system of claim 2, where more than one of the plurality of splayed portions is generally longitudinally aligned another of the plurality of splayed portions.

4. The fiducial deployment system of any preceding claim, where the at least one fiducial comprises a plurality of fiducials, each including a proximal fiducial end and a distal fiducial end, where the proximal end of at least one of the fiducials is immediately adjacent the distal end of another of the fiducials.

5. The fiducial deployment system of any preceding claim, where the at least one splayed portion is comprised by a proximal region of the at least one fiducial.

6. The fiducial deployment system of any preceding claim, where the at least one splayed portion comprises a generally transverse split into a proximal end of the at least one fiducial.

7. The fiducial deployment system of any preceding claim, where the second outer diameter of the at least one fiducial generally is disposed proximal of the first outer diameter of the at least one fiducial.

8. The fiducial deployment system of any preceding claim, where the at least one fiducial comprises a generally cylindrical portion.

9. The fiducial deployment system of any preceding claim, where the needle comprises at least one echogenically-enhanced region.

10. The fiducial deployment system of any preceding claim, where the at least one needle protrusion is provided by a dimple in the tubular cannula body.

11. The fiducial deployment system of any of any preceding claim, wherein a distal end of the fiducial is shaped to ease passage across the at least one protrusion.

## Patentansprüche

1. System (200) zum Einsetzen von Bezugsmarken, umfassend:
eine Nadel (202) mit
einem allgemein röhrenförmigen Kanülenkörper (208), der ein Nadellumen (206) definiert, das zumindest durch einen Längsabschnitt des Kanülenkörpers angeordnet ist, und
einer distalen Nadelendregion, wobei die distale Endregion folgendes umfasst:
eine distale Nadelendöffnung an einem distalen Ende des Nadellumens; und
mindestens einen Vorsprung, der als im Wesentlichen unflexible Sperre (210) ausgelegt ist;
mindestens eine Bezugsmarke (220), umfassend
einen allgemein säulenförmigen Körper (222), der verschiebbar im Nadellumen angeordnet ist und folgendes aufweist:
einen ersten Längsabschnitt (222a) mit einem ersten Außendurchmesser;
einen zweiten Längsabschnitt (222b), der mit einem zweiten Außendurchmesser, der größer als der erste Außendurchmesser ist, gespreizt ist;
und
einen Mandrin (215), der sich durch einen Abschnitt des Nadellumens erstreckt und dazu ausgelegt ist, die mindestens eine Bezugsmarke an dem mindestens einen Vorsprung vorbei und aus der distalen Nadelendöffnung heraus vorzuschieben;
**dadurch gekennzeichnet, dass**:
sich der Vorsprung radial in das Nadellumen erstreckt, um einen begrenzten Innendurchmesser zu bilden, der kleiner als ein Innendurchmesser einer Hauptlänge des Nadellumens ist;
wobei der erste Außendurchmesser der Bezugsmarke im Wesentlichen gleich oder kleiner ist als der begrenzte Innennadeldurchmesser neben dem mindestens einen Vorsprung;
und wobei der zweite Außendurchmesser der Bezugsmarke im Wesentlichen gleich oder kleiner ist als der Innendurchmesser der Hauptlänge des Nadellumens.

2. System zum Einsetzen von Bezugsmarken nach Anspruch 1, wobei der mindestens eine gespreizte Abschnitt der mindestens einen Bezugsmarke eine Vielzahl von gespreizten Abschnitten umfasst.

3. System zum Einsetzen von Bezugsmarken nach Anspruch 2, wobei mehr als einer der Vielzahl von gespreizten Abschnitten allgemein längs mit einem anderen der Vielzahl von gespreizten Abschnitten ausgerichtet sind.

4. System zum Einsetzen von Bezugsmarken nach einem der vorhergehenden Ansprüche, wobei die mindestens eine Bezugsmarke eine Vielzahl von Bezugsmarken umfasst, die jeweils ein proximales Bezugsmarkenende und ein distales Bezugsmarkenende aufweisen, wobei das proximale Ende mindestens einer der Bezugsmarken unmittelbar neben dem distalen Ende einer anderen der Bezugsmarken liegt.

5. System zum Einsetzen von Bezugsmarken nach einem der vorhergehenden Ansprüche, wobei der mindestens eine gespreizte Abschnitt von einer proximalen Region der mindestens einen Bezugsmarke umfasst wird.

6. System zum Einsetzen von Bezugsmarken nach einem der vorhergehenden Ansprüche, wobei der mindestens eine gespreizte Abschnitt einen allgemein quer verlaufenden Schlitz in einem proximalen Ende der mindestens einen Bezugsmarke umfasst.

7. System zum Einsetzen von Bezugsmarken nach einem der vorhergehenden Ansprüche, wobei der zweite Außendurchmesser der mindestens einen Bezugsmarke allgemein proximal vom ersten Außendurchmesser der mindestens einen Bezugsmarke angeordnet ist.

8. System zum Einsetzen von Bezugsmarken nach einem der vorhergehenden Ansprüche, wobei die mindestens eine Bezugsmarke einen allgemein zylindrischen Abschnitt umfasst.

9. System zum Einsetzen von Bezugsmarken nach einem der vorhergehenden Ansprüche, wobei die Nadel mindestens eine echogen verstärkte Oberfläche umfasst.

10. System zum Einsetzen von Bezugsmarken nach einem der vorhergehenden Ansprüche, wobei der mindestens eine Nadelvorsprung von einer Vertiefung im röhrenförmigen Kanülenkörper bereitgestellt wird.

11. System zum Einsetzen von Bezugsmarken nach einem der vorhergehenden Ansprüche, wobei ein distales Ende der Bezugsmarke zur Erleichterung der Passage über den mindestens einen Vorsprung ausgebildet ist.

## Revendications

1. Système de déploiement de repères d'alignement (200), comprenant:
une aiguille (202) comprenant
un corps de canule essentiellement tubulaire (208) qui définit une lumière d'aiguille (206) disposée à travers au moins une partie dans le sens de la longueur du corps de canule; et
une région d'extrémité d'aiguille distale, la région d'extrémité distale comprenant:
une ouverture d'extrémité d'aiguille distale à une extrémité distale de la lumière d'aiguille; et
au moins une protubérance configurée comme une détente sensiblement inflexible (210);
au moins un repère d'alignement colonnaire (220) comprenant:
un corps essentiellement colonnaire (222) disposé de façon coulissante dans la lumière d'aiguille et comprenant:
une première partie dans le sens de la longueur (222a) présentant un premier diamètre extérieur;
une seconde partie dans le sens de la longueur (222b) qui est évasé avec un second diamètre extérieur plus grand que le premier diamètre extérieur; et
un stylet (215) qui s'étend à travers une partie de la lumière d'aiguille et qui est configuré de manière à faire avancer ledit au moins un repère d'alignement au-delà de ladite au moins une protubérance et hors de l'ouverture d'extrémité d'aiguille distale,
**caractérisé en ce que**:
ladite protubérance s'étend radialement dans la lumière d'aiguille de manière à former un diamètre intérieur restreint qui est inférieur à un diamètre intérieur d'une longueur majeure de la lumière d'aiguille;
dans lequel le premier diamètre extérieur du repère d'alignement est sensiblement identique ou inférieur au diamètre d'aiguille intérieur restreint à proximité de ladite au moins une protubérance; et
dans lequel le second diamètre extérieur du repère d'alignement est sensiblement identique ou inférieur au diamètre intérieur de la longueur majeure de la lumière d'aiguille.

2. Système de déploiement de repères d'alignement selon la revendication 1, dans lequel ladite au moins une partie évasée dudit au moins un repère d'alignement comprend une pluralité de parties évasées.

3. Système de déploiement de repères d'alignement selon la revendication 2, dans lequel plusieurs parmi la pluralité de parties évasées sont alignées de façon essentiellement longitudinale avec une autre de la pluralité de parties évasées.

4. Système de déploiement de repères d'alignement selon l'une quelconque des revendications précédentes, dans lequel ledit au moins un repère d'alignement comprend une pluralité de repères d'alignement, qui présentent chacun une extrémité de repère d'alignement proximale et une extrémité de repère d'alignement distale, dans lequel l'extrémité proximale d'au moins un des repères d'alignement est immédiatement adjacente à l'extrémité distale d'un autre des repères d'alignement.

5. Système de déploiement de repères d'alignement selon l'une quelconque des revendications précédentes, dans lequel ladite au moins une partie évasée est constituée par une région proximale dudit au moins un repère d'alignement.

6. Système de déploiement de repères d'alignement selon l'une quelconque des revendications précédentes, dans lequel ladite au moins une partie évasée comprend une fente essentiellement transversale dans une extrémité proximale dudit au moins un repère d'alignement.

7. Système de déploiement de repères d'alignement selon l'une quelconque des revendications précédentes, dans lequel le second diamètre extérieur dudit au moins un repère d'alignement est essentiellement disposé à proximité du premier diamètre extérieur dudit au moins un repère d'alignement.

8. Système de déploiement de repères d'alignement selon l'une quelconque des revendications précédentes, dans lequel ledit au moins un repère d'alignement comprend une partie essentiellement cylindrique.

9. Système de déploiement de repères d'alignement selon l'une quelconque des revendications précédentes, dans lequel l'aiguille comprend au moins une région renforcée échonégiquement.

10. Système de déploiement de repères d'alignement selon l'une quelconque des revendications précédentes, dans lequel ladite au moins une protubérance d'aiguille est formée par un creux dans le corps de canule tubulaire.

11. Système de déploiement de repères d'alignement selon l'une quelconque des revendications précédentes, dans lequel une extrémité distale du repère d'alignement est configurée de manière à faciliter le passage à travers ladite au moins une protubérance.
